# EUROPEAN PATENT SPECIFICATION

(11) **EP 2 734 129 B1**
(45) Date of publication and mention of the grant of the patent: **09.01.2019**
(21) Application number: 12867745.7
(22) Date of filing: 29.02.2012
(51) Int. Cl.: A61B 17/24, A61M 25/00, A61B 17/00, A61B 90/00

(54) **APPARATUS FOR FACILITATING INTRANASAL TREATMENT OF A PATIENT**
VORRICHTUNG ZUR INTRANASALEN BEHANDLUNG EINES PATIENTEN
APPAREIL POUR FACILITER LE TRAITEMENT INTRANASAL D'UN PATIENT

(30) Priority: 10.02.2012 US 201213371288
(43) Date of publication of application: 28.05.2014
(73) Proprietor: Dolor Technologies, LLC, Carson City, Nevada 89706 (US)
(72) Inventor: ELDREDGE, Stephen, South Jordan, Utah 84095 (US); ERICKSEN, Corey, Fruit Heights, Utah 84037 (US); O'DRISCOLL, Jeffrey, Salt Lake City, Utah 84108 (US); PIPKIN, William, Orem, Utah 84097 (US)
(74) Representative: Alt, Michael
(86) International application number: PCT/US2012/027138
(87) International publication number: WO 2013/119258

(56) References cited:
- EP-A1- 2 106 814
- WO-A1-2009/124192
- US-A- 5 830 189
- US-A1- 2004 015 068
- US-A1- 2007 021 648
- US-A1- 2010 030 187
- US-A1- 2010 057 048
- US-A1- 2010 057 048
- US-A1- 2010 121 336
- US-A1- 2011 152 838
- US-B2- 7 632 243

## Description

### FIELD OF ART

This subject matter relates to autonomic and nociceptive nerve blockade and more particularly relates to blockade of sphenopalatine/pterygopalatine ganglia. Specifically, the instant subject matter presents apparatus, which, in use extenuate, mitigate, and ameliorate pain attributed to numerous indications.

### BACKGROUND OF THE ART

Autonomic pain is a type of nerve pain that arises due to abnormalities in the function of the autonomic nervous system. With autonomic pain, an abnormality in a group of nerves called a ganglion causes pain to an organ or body region. To treat autonomic-mediated pain physicians can block a ganglion with the injection or application of medication to a specific area of the body. To therapeutically treat acute pain a physician injects or applies a local anesthetic to the affected neuronal ganglion. This type of treatment may be referred to as a nerve block.

In 1908, Greenfield Sluder M.D., published his article, "The role of the sphenopalitine ganglion in nasal headaches," in the New York Medical Journal*.* He advocated using a long needle through the side of the face to inject cocaine into the sphenopalitine ganglion (SPG) to treat certain severe recurrent headaches. More than a century of medical science has validated Sluder's basic premise: that sphenopalitine ganglion blockade (SPGB) is a valuable tool in headache management. However, prior to the instant teachings, a paucity of tools exists for the same.

The SPG is a collection of nerve cells resting just beneath the thin tissues that line the back of the nasal passage. Because of the neural connections that pass through it, the SPG plays an essential role in various types of headaches. Temporary interruption of impulse conduction through the SPG can often abort headaches and sometimes provide long-term relief to headache sufferers.

Other conditions shown to respond to SPGB in published literature include: trigeminal neuralgia, dental pain, post-partum neck and back pain, complex regional pain syndrome, herpes zoster (Shingles), tempromandibular joint (TMJ) pain and primary hyperhidrosis, among other indications, as discussed herein.

Aside from the personal pain suffered by those who experience recurrent severe headaches, the staggering financial cost to society is difficult to estimate or comprehend. Just for the 30 million migraine sufferers in the United States, annual direct medical costs are estimated to exceed $12B, with lost productivity costing employers an additional $12B. Those figures do not include the rest of the world or the two dozen other types of cephalgia found in the World Health Organization's headache classification scheme.

An estimated 4-5% of the population suffers from chronic daily headaches which, by definition, impact a person's ability to function for at least 15 days a month for at least 3 months. Of those patients, 30% are managed with relatively inexpensive medications; 17% require pharmacologic regimens exceeding $500/month; and more than half continue to suffer a virtual failure of modern medicine.

Any intervention that reduces the incidence or duration of headaches has the potential to dramatically reduce personal suffering and to save patients, insurance companies and governments enormous sums of money. The SphenoCath™ brand of catheter system offers a simple, safe, inexpensive intervention, as explained throughout below.

The SPG/pterygopalatine ganglia is a neuronal structure located principally in the center of the head in the pterygopalatine fossa posterior to the middle turbinate. The SPG/pterygopalatine ganglia comprises the largest cluster of sympathetic neurons in the head outside of the brain. The SPG/pterygopalatine ganglia interfaces and directs nerve impulses to the majority of the head's autonomic or parasympathetic pathways. Therefore, any abnormality or injury to this structure may cause severe pain. A nerve block of the SPG/pterygopalatine ganglia may relieve a variety of painful conditions ranging from headache to lower back pain. Additionally, other disease processes such as headache disorders and other neurological conditions can be arrested, or improved by local anesthetic blockade, and/or other pharmacological augmentation or mechanical alteration of the SPG/pterygopalatine ganglia and surrounding structures.

Unfortunately, because of the anatomical position of the SPG/pterygopalatine ganglia, the structure is very difficult to block with a local anesthetic solution. The anatomical location of the SPG/pterygopalatine ganglia is dangerously close to many vital and delicate mid brain structures. Although direct needle placement can be employed under fluoroscopic guidance to administer anesthetic to the SPG/pterygopalatine ganglia, most practitioners will not attempt the procedure due to the technical difficulty and extreme dangers of an aberrant needle placement.

Accessing the SPG/pterygopalatine recess to treat the SPG/pterygopalatine ganglia with a conventional device is difficult in that a conventional devices typically do not include a curvature for accessing the sphenopalatine/pterygopalatine recess. Further, even if a conventional needle were curved to access the sphenopalatine/pterygopalatine recess, once the curved needle were inserted into the patient's nasal cavity, the physician or other medical professional would not be able to identify the direction of the curve of the needle. Without fluoroscopic guidance, an insertion end of the needle may contact and/or damage the vital and delicate mid brain structures. To date, this limitation has limited both service-providers and patients from being involved to a large extent.

US 2011/152838 A1 discloses a device to access the SPG of a patient.

### SUMMARY

From the foregoing discussion, it should be apparent that a need exists for a step change, namely an apparatus for facilitating intranasal treatment of a patient which can be done safely, in due course, by those other than the top half-dozen medical professionals who can do so successfully. Beneficially, such an apparatus would administer medication directly to the sphenopalatine/pterygopalatine ganglia.

The present subject matter has been developed in response to the present state of the art, and in particular, in response to the problems and needs in the art that have not yet been fully solved by currently available intranasal treatment apparatus. Accordingly, the present subject matter has been developed to provide an apparatus, for intranasal treatment of a patient that overcome many or all of the above-discussed shortcomings in the art. The invention provides an apparatus according to claim 1. Further embodiments of the invention are provided in the dependent claims.

The apparatus to facilitate intranasal treatment of a patient's SPG/pterygopalatine recess includes a sheath hub, a catheter hub, an arresting element, and an engagement element. The sheath hub has an exterior surface opposing an interior surface. The interior surface defines a catheter hub receiving space. The catheter hub is slideably received within the catheter hub receiving space and is positionable along a longitudinal axis of the sheath hub. The arresting element is positioned on one of the catheter hub and the sheath hub and the engagement element is positioned on the other of the catheter hub and the sheath hub. The arresting element continuously engages the engagement element when the catheter hub is positioned along the longitudinal axis of the sheath hub. Engagement between the arresting element and the engagement element prevents rotation of the sheath hub with respect to the catheter hub.

The apparatus, in one embodiment, includes a catheter and a rotational orientation indicator. The catheter is coupled to the catheter hub and at least a portion of the catheter includes an intrinsic curvature. The rotational orientation indicator identifies the rotational orientation of the intrinsic curvature of the catheter. In certain embodiments, the rotational orientation indicator comprises a raised ridge extending longitudinally along at least one of the sheath hub and the catheter hub. In other embodiments, the rotational orientation indicator only provides a visual indication of the rotational orientation of the intrinsic curvature of the catheter.

The catheter includes an insertion end and a coupling end. The insertion end includes the intrinsic curvature with respect to a longitudinal axis of the catheter and, in an embodiment, the rotational orientation indicator identifies the rotational orientation of the intrinsic curvature of the catheter.

The apparatus includes a sheath coupled to the sheath hub and a catheter coupled to the catheter hub and received within the sheath. The catheter hub is positionable between an inserted position and an extended position. With the catheter hub positioned in the extended position, the sheath straightens the intrinsic curvature of the catheter.

The catheter, in some embodiments, includes an insertion end having a tip that is curved such that the tip is rounded. The sheath includes an introduction end having an outermost edge that is sloped to a vertex. In such an embodiment, when the catheter hub is positioned in the extended position, the vertex aligns with a beginning of the curve of the rounded tip of the catheter. In certain embodiments, a transition between the vertex and the beginning of the curve of the rounded tip of the catheter is continuous when the catheter hub is positioned in the extended position. In one embodiment, a tip of the insertion end of the catheter is bulbous.

The arresting element, in certain embodiments, is a flange coupled to and extending perpendicularly from either an outer surface of the catheter hub or an interior surface of the sheath hub. In such an embodiment, the engagement element is a recess extending longitudinally along the other of the interior surface of the sheath hub or the outer surface of the catheter hub. The flange is positioned within and travels along the recess when the catheter hub is repositioned along the longitudinal axis of the sheath hub.

In certain embodiments, the apparatus includes a stopping element coupled to either the catheter hub or the sheath hub. The stopping element is configured to engage the arresting element to stop the catheter hub from being removed from the catheter hub receiving space. In one embodiment, the stopping element is configured to align the vertex of the sheath with the beginning of the curve of the rounded tip of the catheter when the catheter hub is positioned in the extended position.

Reference throughout this specification to features, advantages, or similar language does not imply that all of the features and advantages that may be realized with the present subject matter should be or are in any single embodiment of the subject matter. Rather, language referring to the features and advantages is understood to mean that a specific feature, advantage, or characteristic described in connection with an embodiment is included in at least one embodiment of the present subject matter. Thus, discussion of the features and advantages, and similar language, throughout this specification may, but do not necessarily, refer to the same embodiment.

Furthermore, the described features, advantages, and characteristics of the subject matter may be combined in any suitable manner in one or more embodiments. One skilled in the relevant art will recognize that the subject matter may be practiced without one or more of the specific features or advantages of a particular embodiment. In other instances, additional features and advantages may be recognized in certain embodiments that may not be present in all embodiments of the subject matter.

These features and advantages of the present subject matter will become more fully apparent from the following description and appended claims, or may be learned by the practice of the subject matter as set forth hereinafter.

### BRIEF DESCRIPTION OF THE DRAWINGS

In order that the advantages of the subject matter will be readily understood, a more particular description of the subject matter briefly described above will be rendered by reference to specific embodiments that are illustrated in the appended drawings. Understanding that these drawings depict only typical embodiments of the subject matter and are not therefore to be considered to be limiting of its scope, the subject matter will be described and explained with additional specificity and detail through the use of the accompanying drawings, in which:
Figure 1 is a cutaway view illustrating one embodiment of the facial anatomy of a patient upon which the apparatus of the present invention may be employed;
Figure 2 is a cutaway view illustrating a prior art method of treating headaches;
Figure 3 is a cutaway view illustrating a prior art method of treating headaches;
Figure 4A is a perspective view illustrating one embodiment of an apparatus for facilitating intranasal treatment of a patient's sphenopalatine/pterygopalatine recess with a catheter hub positioned in an inserted position within a sheath hub (ready for medication delivery) in accordance with the present subject matter;
Figure 4B is a perspective view illustrating one embodiment of an apparatus for facilitating intranasal treatment of a patient's sphenopalatine/pterygopalatine recess with a catheter hub positioned in an extended position within a sheath hub (ready for device insertion) in accordance with the present subject matter;
Figure 5A is a top view illustrating one embodiment of an apparatus for facilitating intranasal treatment of a patient's sphenopalatine/pterygopalatine recess with a catheter hub positioned in an extended position within a sheath hub (ready for device insertion) in accordance with the present subject matter;
Figure 5B is a side cutaway view illustrating one embodiment of an apparatus for facilitating intranasal treatment of a patient's sphenopalatine/pterygopalatine recess with a catheter hub positioned in an extended position within a sheath hub (ready for device insertion) in accordance with the present subject matter;
Figure 6A is a top view illustrating one embodiment of an apparatus for facilitating intranasal treatment of a patient's sphenopalatine/pterygopalatine recess with a catheter hub positioned in an inserted position within a sheath hub (ready for medication delivery) in accordance with the present subject matter;
Figure 6B is a side cutaway view illustrating one embodiment of an apparatus for facilitating intranasal treatment of a patient's sphenopalatine/pterygopalatine recess with a catheter hub positioned in an inserted position within a sheath hub (ready for medication delivery) in accordance with the present subject matter;
Figure 7 is a perspective view illustrating one embodiment of a catheter hub in accordance with the present subject matter;
Figure 8A is a top view illustrating one embodiment of a catheter hub and a catheter in accordance with the present subject matter;
Figure 8B is a side cutaway view illustrating one embodiment of a catheter hub and a catheter in accordance with the present subject matter;
Figure 9A is an end view depicting one embodiment of a catheter hub taken in the direction of the treatment receiving end of the catheter hub in accordance with the present subject matter;
Figure 9B is an end view depicting one embodiment of a catheter hub taken in the direction of the treatment delivery end of the catheter hub in accordance with the present subject matter;
Figure 10A is a top view illustrating one embodiment of a sheath hub and a sheath in accordance with the present subject matter;
Figure 10B is a side cutaway view illustrating one embodiment of a sheath hub and a sheath in accordance with the present subject matter;
Figure 11 is an enlarged view of an area of the sheath hub which includes the stopping element in accordance with the present subject matter;
Figure 12 is an end view depicting one embodiment of the sheath hub taken in the direction of the sheath receiving end of the sheath hub in accordance with the present subject matter;
Figure 13A is a cutaway view illustrating one embodiment of an introduction end of the sheath and an insertion end of the catheter with a vertex of the sheath aligning with a beginning of a curve of the insertion end of the catheter;
Figure 13B is a cutaway view illustrating one embodiment of an introduction end of the sheath and an insertion end of the catheter with a vertex of the sheath out of alignment with a beginning of a curve of the insertion end of the catheter; and,
Figure 13C is a cutaway view illustrating one embodiment of an introduction end of the sheath and an insertion end of the catheter with a vertex of the sheath out of alignment with a beginning of a curve of the insertion end of the catheter.

### DETAILED DESCRIPTION

The present inventors have discovered that enhanced systems can enable those who are not licensed physicians (non-surgically trained operators with training by licensed healthcare professionals) to address SPG issues with unexpected rates of success.

The SphenoCath™ brand of medical device, when used as designed, allows any practitioner the directional control necessary to deliver an appropriate dose of medication safely and painlessly to the SPG in an office setting without needles, sedation or narcotics.

Reference throughout this specification to "one embodiment," "an embodiment," or similar language means that a particular feature, structure, or characteristic described in connection with the embodiment is included in at least one embodiment of the present subject matter. Thus, appearances of the phrases "in one embodiment," "in an embodiment," and similar language throughout this specification may, but do not necessarily, all refer to the same embodiment.

Furthermore, the described features, structures, or characteristics of the subject matter may be combined in any suitable manner in one or more embodiments. In the following description, numerous specific details are provided for a thorough understanding of embodiments of the subject matter. One skilled in the relevant art will recognize, however, that the subject matter may be practiced without one or more of the specific details, or with other methods, components, materials, and so forth. In other instances, well-known structures, materials, or operations are not shown or described in detail to avoid obscuring aspects of the subject matter.

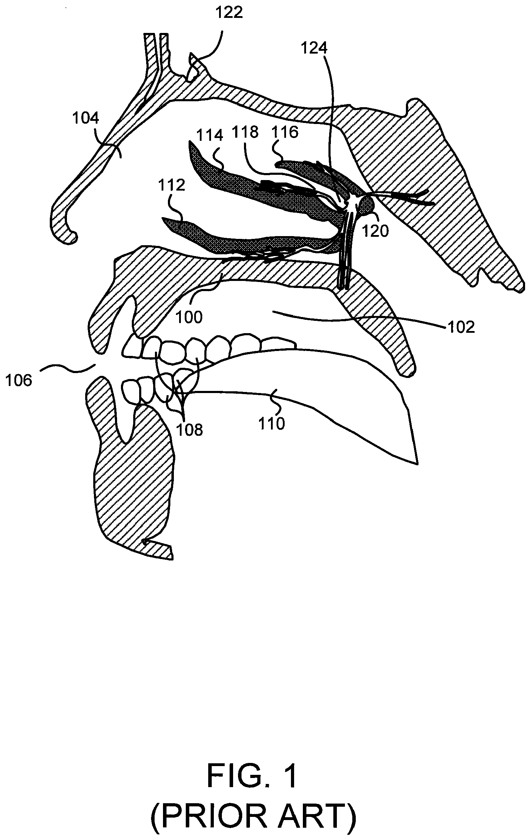

Figure 1 is an illustration of one environment in which the present devices and system operate. In particular, Figure 1 depicts a cutaway view of the anatomical features of a typical human nasal cavity. One skilled in the art will recognize that certain anatomical features and structures of the human nasal cavity have been omitted to avoid obscuring the structures relevant to the practice of the current subject matter. To help orient the reader, the mouth 106 is illustrated with teeth 108 and tongue 110. The anatomical structures relevant to one practice of the current subject matter include the palate 100 which separates the oral cavity 102 from the nasal cavity 104, the inferior sinus turbinate 112, the middle sinus turbinate 114 and the superior sinus turbinate 116 as well as the nasal bone 122. The middle sinus turbinate 114 and superior sinus turbinate 116 define the sphenopalatine/pterygopalatine recess 118. Deep within the sphenopalatine/pterygopalatine recess 118 at the posterior 124 of the sphenopalatine/pterygopalatine recess 118 lies the sphenopalatine/pterygopalatine ganglia 120.

One skilled in the art will recognize that the medical community is not uniform in the terminology with regard to the sphenopalatine or pterygopalatine ganglia. Certain practitioners use sphenopalatine while others use pterygopalatine. Therefore, the present description will refer to the ganglion labeled 120 as the SPG/pterygopalatine ganglia 120. Similarly, the recess labeled 118 will be referred to as the SPG/pterygopalatine recess 118. However, this terminology is in no way limiting on the structure for which the present subject matter is intended. Where practitioners or scientist differentiate between the sphenopalatine ganglion or the pterygopalatine ganglia, the present disclosure will be understood to apply to either structure.

The sphenopalatine/pterygopalatine ganglia 120 is a neuronal structure located principally in the center of the head in the pterygopalatine fossa posterior to the middle turbinate 114. The sphenopalatine/pterygopalatine ganglia 120 comprises the largest cluster of sympathetic neurons in the head outside of the brain. The sphenopalatine/pterygopalatine ganglia 120 interfaces and directs nerve impulses to the majority of the head's autonomic or parasympathetic pathways. Therefore, any abnormality or injury to this structure may cause severe pain. A nerve block of the sphenopalatine/pterygopalatine ganglia 120 may be effective in relieving a variety of pain conditions ranging from headache to lower back pain. Additionally, other disease processes such as headache disorders and other neurological conditions can be arrested, or improved by local anesthetic blockade, and/or other pharmacological augmentation or mechanical alteration of the sphenopalatine/pterygopalatine ganglia 120 and surrounding structures.

Unfortunately, because of the anatomical position of the sphenopalatine/pterygopalatine ganglia 120, the structure may be very difficult to block with a local anesthetic solution using some commonly practiced techniques. The anatomical location of the sphenopalatine/pterygopalatine ganglia 120 is dangerously close to many vital and delicate mid-brain structures. Although direct needle placement can be employed under fluoroscopic guidance to administer anesthetic to the sphenopalatine/pterygopalatine ganglia 120, most practitioners will not attempt the procedure due to the technical difficulty and extreme dangers of an aberrant needle placement.

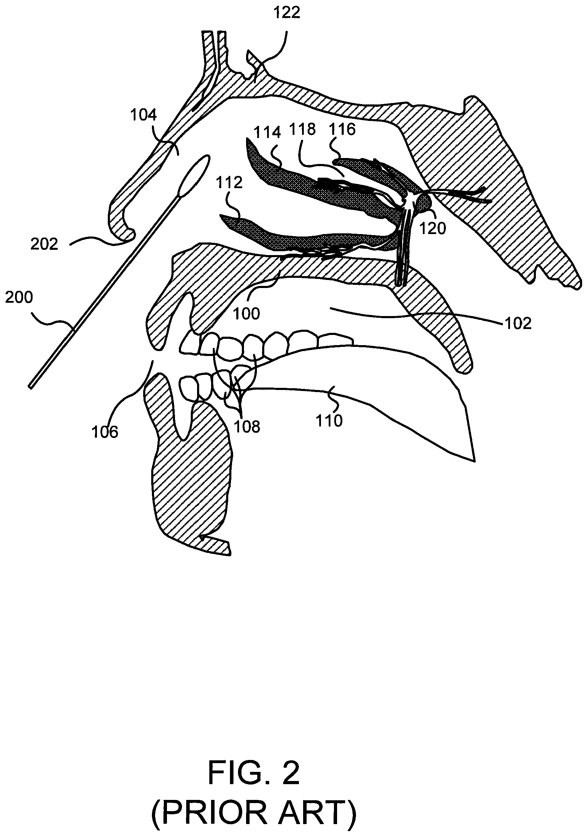

As shown in the prior art illustration depicted in Figure 2, the sphenopalatine/pterygopalatine ganglia 120 lies deep within the sphenopalatine/pterygopalatine recess 118. Conventional methods undertaken by pain specialists, neurologists, and neurosurgeons, include the use of an 20.32 centimetre (eight inch) cotton-tipped applicator 200 saturated with a local anesthetic. Because a cotton-tipped applicator 200 is used, the procedure is referred to as the "Q-tip" procedure. The cotton-tipped applicator 200 is soaked in a vial of concentrated local anesthetic solution. In certain examples the anesthetic solution is lidocaine, cocaine, etidocaine or prilocaine, or other non-specified local anesthetic agents. The cotton-tipped applicator 200 is then advanced into the nostril 202 and through the nasal cavity 104. To reach the sphenopalatine/pterygopalatine ganglia 120 in the sphenopalatine/pterygopalatine recess 118, the cotton-tipped applicator 200 must be advanced into the nasal cavity 104 past the middle sinus turbinate 114 and into the sphenopalatine/pterygopalatine recess 118.

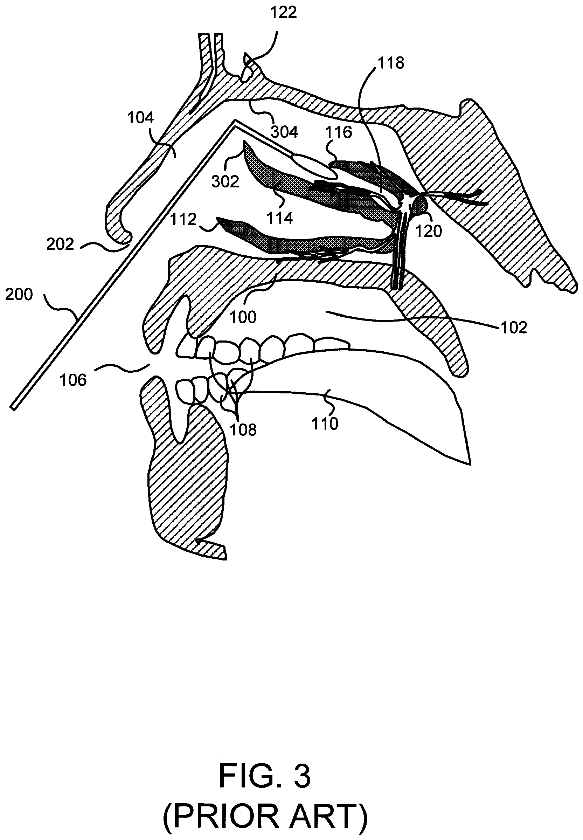

Figure 3 illustrates the tortuous path the cotton-tipped applicator 200 of the prior art must traverse to reach the sphenopalatine/pterygopalatine recess 118. To perform the procedure the patient is placed in a supine position. The cotton-tipped applicator 200 is soaked in a vial of concentrated local anesthetic solution or other pharmacologic agent. The physician then inserts the cotton-tipped applicator 200 into the patient's nostril 202 and through the nasal cavity 104. Advancing the straight, rigid cotton-tipped applicator 200 into the sphenopalatine/pterygopalatine recess 118 can be difficult and painful for the patient as the cotton-tipped applicator 200 must be inserted almost parallel to the patient's face to clear the anterior ridge 302 of the middle sinus turbinate 114. The cotton-tipped applicator 200 must then make an almost 90° bend to avoid the inferior surface 304 of the nasal bone 122 and access the sphenopalatine/pterygopalatine recess 118. The cotton-tipped applicator 200 is left in the patient's sphenopalatine/pterygopalatine recess 118 for approximately 20 minutes to allow diffusion of the local anesthetic or other pharmacologic agent through the sinus mucosa to modulate the sphenopalatine/pterygopalatine ganglia 120 to temporarily block or permanently ablate nerve transmission.

The use of a straight and rigid cotton-tipped applicator 200 that must make some fairly tortuous directional changes around some very sensitive, richly vascular, friable, highly innervated structures complicates the procedure to the point that many practitioners will not attempt it. Known complications include extreme patient discomfort, nosebleeds and the complications associated with nosebleeds including venous-irritating nuisances, arterial hemorrhaging, aspiration, hematochezia or even death. Other complications include local anesthetic toxicity, seizure, iatrogenic foreign bodies such as a broken cotton-tipped applicator 200, sinus mucosal tears and infection.

Anesthetic blockade of any neuronal structure requires direct physical interaction between the anesthetic solution and the targeted tissue. Therefore, to work, the cotton-tipped applicator 200 must deliver the anesthetic solution directly to the sphenopalatine/pterygopalatine ganglia 120. The correct placement of the cotton-tipped applicator 200 is technically challenging and many practitioners simply miss the desired structure, the sphenopalatine/pterygopalatine ganglia 120 when attempting to perform the procedure. To help make the complicated bend required to reach the sphenopalatine/pterygopalatine recess 118 many practitioners will soak the top 5.08 centimetres (2 inches) of the cotton-tipped applicator 200 and manipulate the stem to render it flexible so that the patient is less agitated and bleeding risks are lessened. Even with a flexible cotton-tipped applicator 200 the procedure is difficult. Common failure placements include the inferior surface 304 of the nasal bone 122 and the anterior ridge 302 of the middle sinus turbinate 114. When the cotton-tipped applicator 200 is misplaced, a "wring-out" effect may occur wherein the anesthetic is wrung out of the cotton-tipped applicator before it is delivered to the sphenopalatine/pterygopalatine ganglia 120 resulting in an ineffective procedure. Further, as discussed above, the rich vascular and neuronal structure of the nasal cavity 104 makes any misplacement of the cotton-tipped applicator 200 both dangerous and painful.

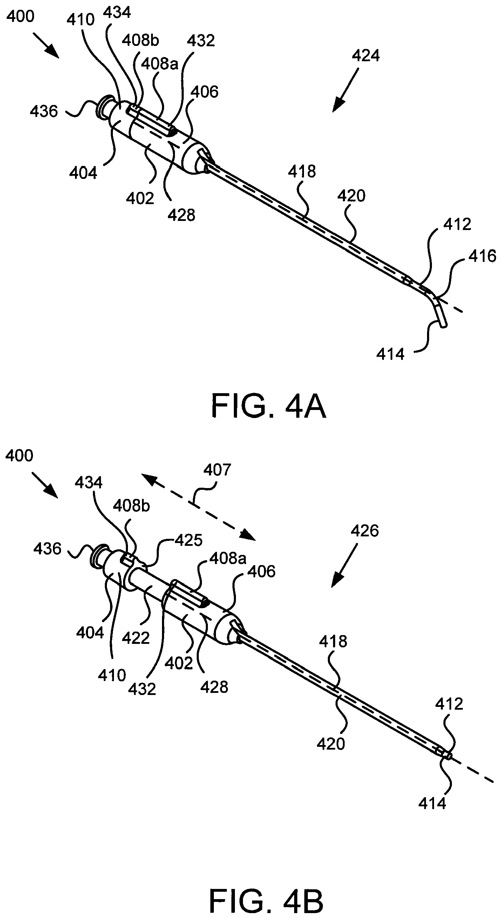

Figure 4A depicts a perspective view of one embodiment of an apparatus 400 for facilitating intranasal treatment of a patient's sphenopalatine/pterygopalatine recess 118. The apparatus 400 includes the sheath hub 402, the catheter hub 404, a catheter 412, and a sheath 420. The apparatus 400 of Figure 4A is depicted with a catheter hub 404 positioned in an inserted position 424 within a sheath hub 402. Figure 4B depicts a perspective view of one embodiment of the apparatus 400 of Figure 4A with the catheter hub 404 positioned in an extended position 426 within the sheath hub 402.

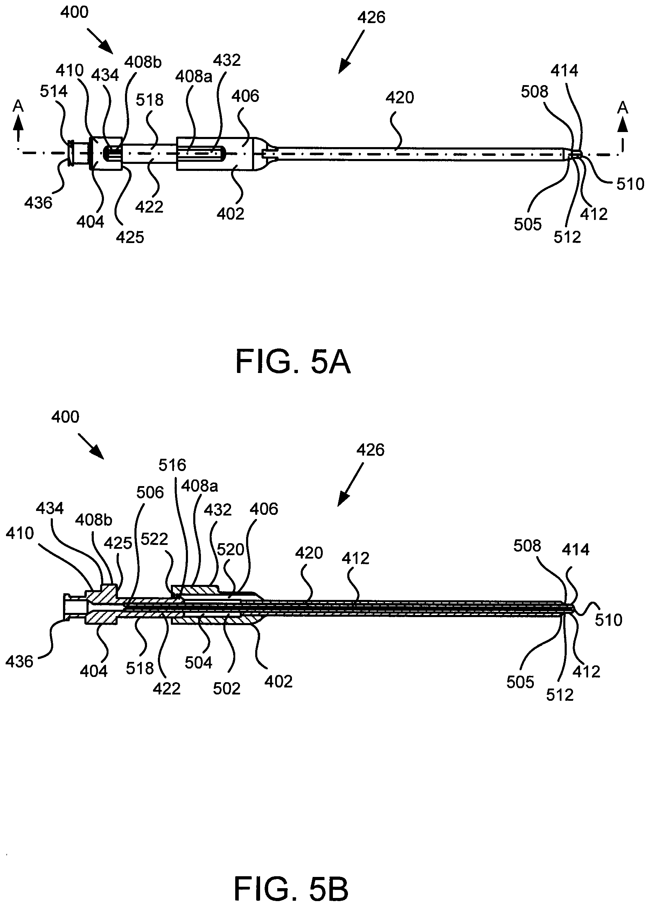

The sheath hub 402 includes an exterior surface 406 that opposes an interior surface 502 (Figure 5B). The interior surface 502 of the catheter hub 404 defines a catheter hub receiving space 504 (Figure 5B). At least a portion 422 of the catheter hub 404 is received within the catheter hub receiving space 504 and the catheter hub 404 is repositionable along a longitudinal axis 428 of the sheath hub 402. The catheter hub 404 is repositionable between an inserted position 424 as illustrated in Figure 4A and an extended position 426 as illustrated in Figure 4B.

The catheter hub 404 includes a stopping surface 425. When the catheter hub 404 is fully inserted into the catheter hub receiving space 504, the stopping surface 425 of the catheter hub 404 contacts the sheath hub 402 to arrest further insertion of the catheter hub 404 within the catheter hub receiving space 504. With the catheter hub 404 fully positioned within the catheter hub receiving space 504 to the point where the stopping surface 425 contacts the sheath hub 402, the catheter hub 404 may be considered to be positioned in the fully inserted position 424 with the internal catheter being fully extended beyond the distal tip of the sheath. As the catheter hub 404 is withdrawn from within the catheter hub receiving space 504 in the direction indicated by arrows 407, the catheter hub 404 may be considered to be positioned in a fully extended position 426.

In the depicted embodiments, the profile of the sheath hub 402 and the profile of the catheter hub 404 are substantially circular. In other embodiments, the sheath hub 402 and the catheter hub 406 may have a triangular profile, a square profile, a rectangular profile, an octagonal profile, an oblong profile, or a profile having any other geometric shape.

A catheter 412 is coupled to the catheter hub 404 at a coupling end 506 (see Figure 5B) of the catheter 412. An insertion end (distal tip) 414 of the catheter 412 includes an intrinsic curvature 416 with respect to a longitudinal axis 418 of the catheter 412. The insertion end 414 of the catheter 412 is disposed opposite the coupling end 506 of the catheter 412. The intrinsic curvature 416 of the insertion end 414 of the catheter 412 causes the insertion end 414 of the catheter 412 to bend when advanced beyond the distal tip of the sheath 420. When the apparatus 400 is inserted into the patient's nasal cavity 104 the intrinsic curvature 416 of the insertion end 414 is retracted into the sheath 420 until the physician or other medical professional advances the catheter beyond the distal tip of the sheath 420 into the space directly above the patient's sphenopalatine/pterygopalatine recess 118 without contacting the delicate surrounding structures of the patient's nasal cavity 104.

When the catheter 412 is first inserted into the patient's nasal cavity 104, the catheter 412 must travel in a relatively straight path past the anterior ridge 302 of the middle sinus turbinate 114 to access the patient's sphenopalatine/pterygopalatine recess 118. Therefore, upon insertion into the patient's nasal cavity, the catheter 412 should be relatively straight. In order to straighten the intrinsic curvature 416 of the insertion end 414 of the catheter 412, the catheter 412 is received within a sheath 420 which has a sufficient structural rigidity to straighten the intrinsic curvature 416 of the insertion end 414 of the catheter 412. The sheath 420 is coupled to the sheath hub 402 and the catheter 412 is retracted within the sheath 420.

Because the catheter 412 is coupled to the catheter hub 404 and the sheath 420 is coupled to the sheath hub 402, when the catheter hub 404 is positioned in the extended position 426 the catheter 412 is withdrawn into the sheath 420 as illustrated in Figure 4B. With the catheter 412 withdrawn into the sheath 420, the structural rigidity of the sheath 420 straightens the intrinsic curvature 416 of the insertion end 414 of the catheter 412 allowing the physician or other medical professional to manipulate the insertion end 414 of the catheter 412 past the anterior ridge 302 of the middle sinus turbinate 114.

Once the insertion end 414 of the catheter 412 has passed the anterior ridge 302 of the middle sinus turbinate 114, the physician or other medical professional can advance the catheter hub 404 to the inserted position 424. With the catheter hub 404 repositioned in the inserted position 424 the intrinsic curvature 416 of the insertion end 414 of the catheter 412 is not positioned within the sheath 420 and is therefore not straightened by the sheath 420. The intrinsic curvature 416 of the insertion end 414 of the catheter 412 causes the insertion end 414 of the catheter 412 to bend. The bend in the catheter 412 allows the physician or other medical professional to direct the insertion end 414 of the catheter 412 into the patient's sphenopalatine/pterygopalatine recess 118 where the physician or other medical professional can deliver treatment to the patient's sphenopalatine/pterygopalatine ganglia 120.

While the present discussion is directed to access of the sphenopalatine/pterygopalatine recess 118 to treat the sphenopalatine/pterygopalatine ganglia 120, one of skill in the art will recognize that the apparatus 400 may be used to access other areas of a patient. For example, the apparatus 400 may be used by a physician or other medical professional to position the insertion end 414 of the catheter 412 in any area on the patient that is not linear disposed with reference to the entry point. Examples of such areas may include a patient's aural cavity, veins, arteries, etc.

In certain embodiments, the treatment delivered may be a dispensing of a nerve blockage to the sphenopalatine/pterygopalatine ganglia 120 through the catheter 412. In other embodiments, the catheter 412 may include an electrode configured to deliver an electrical stimulation to the sphenopalatine/pterygopalatine ganglia 120. One of skill in the art will recognize other medical treatments may be delivered to the sphenopalatine/pterygopalatine ganglia 120.

When the physician or other medical professional inserts the insertion end of the catheter 412 into the patient's nasal cavity, the physician's or other medical professional's view of the insertion end 414 of the catheter 412 may be obstructed by the surrounding structure of the patient's nose. Accordingly, the physician or other medical professional cannot see the orientation of the intrinsic curvature 416 or insertion end 414 of the catheter 412 to determine whether or not the intrinsic curvature 416 is oriented in a position to advance the insertion end 414 of the catheter 412 into the patient's sphenopalatine/pterygopalatine recess 118. To aid the physician or other medical professional in advancing the insertion end 414 of the catheter 412 into the patient's sphenopalatine/pterygopalatine recess 118, in certain embodiments, the apparatus 400 includes a rotational orientation indicator 408. In one embodiment, the rotational orientation indicator 408 identifies a rotational orientation of the intrinsic curvature 416 of the catheter 412 to aid the physician or other medical professional in determining the orientation of the insertion end 414 of the catheter. In certain embodiments, the rotational orientation indicator 408 may be a visual indicator such as a line, a dot, or other indication, positioned on an exterior surface 406 of the sheath hub 402, an exterior surface 410 of the catheter hub 404, or both.

In the embodiment illustrated in Figures 4A and 4B, the rotational orientation indicator 408 includes a first rotational orientation indicator 408a positioned on an exterior surface 406 of the sheath hub 402 and a second rotational orientation indicator 408b positioned on an external surface 410 of the catheter hub 404. In other embodiments, only one of the sheath hub 402 or the catheter hub 404 includes a rotational orientation indicator 408.

In one embodiment, the rotational orientation indicator 408a on the sheath hub 402 is a raised ridge 432 that extends longitudinally along at least a portion of the sheath hub 402. The raised ridge 432 extends substantially perpendicularly from the exterior surface 406 of the sheath hub 402 to provide a physician or other medical professional tactile feedback as to the orientation of the intrinsic curvature 416 of the catheter 412. Thus, when the physician or other medical professional advances the insertion end 414 of the catheter 412 into a patient's nasal cavity 104, the physician or other medical professional can focus their visual attention on other factors that may affect the procedure such as the depth of the catheter 412 within the patient's nasal cavity 104.

In certain embodiments, the rotational orientation indicator 408b on the catheter hub 404 may also be a raised ridge 434. In such an embodiment, the raised ridge 434 extends longitudinally along at least a portion of the catheter hub 404. The raised ridge 432 extends substantially perpendicularly from the exterior surface 410 of the catheter hub 404 to provide a physician or other medical professional tactile feedback as to the orientation of the intrinsic curvature 416 of the catheter 412. In embodiments where both the sheath hub 402 and the catheter hub 404 include raised ridges 432 and 434 that act as rotational orientation indicators 408, the physician or other medical professional can determine the orientation of the intrinsic curvature 416 of the catheter 412 regardless of which component (the catheter hub 404 or the sheath hub 402) the physician or other medical professional is manipulating.

In certain embodiments, the raised ridges 432 and 434 on the sheath hub 402 and the catheter hub 404 are aligned along the same axis. In other embodiments, the raised ridge 432 on the sheath hub 402 may be offset from the raised ridge 434 on the catheter hub 404. In yet another embodiment, as discussed above, only one of the sheath hub 402 or the catheter hub 404 includes a rotational orientation indicator 408. In such an embodiment, the apparatus 400 may include either raised ridge 432 on the exterior surface 406 of the sheath hub 402 or raised ridge 434 on the exterior surface 410 of the catheter hub 404.

In one embodiment, the apparatus 400 includes a treatment receiving port 436 that receives a medicinal treatment. For example, in certain embodiments, the treatment receiving port 436 is coupleable to a syringe or other medication delivery device. The treatment receiving port 436 is communicable in fluid communication with the catheter 412 such that medication can be delivered through the catheter 412 to the sphenopalatine/pterygopalatine ganglia 120.

In other embodiments, other treatment delivery devices may be coupled to the treatment receiving port 436. For example, in one embodiment, the treatment delivery device may include an electrical stimulation device configured to transmit an electrical current to the apparatus 400. In such an embodiment, the catheter 412 may include an electrical conduit that conducts a flow of electricity from the treatment receiving port 436 to the insertion end 414 of the catheter 412. An electrode positioned on the insertion end 414 of the catheter 412 delivers the electrical current to the patient.

Figure 5A depicts a top view of one embodiment of an apparatus 400 for facilitating intranasal treatment of a patient's sphenopalatine/pterygopalatine recess 118. In the embodiment depicted Figure 5A, the catheter hub 404 is positioned in the extended position 426. In certain embodiments, the sheath 420 includes an introduction end 505 that is sloped to a vertex 508 such that the entire introduction end 505 of the sheath 420 forms a smooth slope without any edges to catch on the tissue of the patient's nasal cavity 104.

In one embodiment, the insertion end 414 of the catheter 412 is curved such that a tip 510 of the insertion end 414 of the catheter 412 is rounded. By including a rounded tip 510 on the insertion end 414 of the catheter 412, the physician or other medical professional is less likely to catch or snag the delicate tissue of the patient's nasal cavity 104 with the insertion end 414 of the catheter 412. As further described below, in certain embodiments, when the catheter hub 404 is positioned in the extended position 426, the vertex 508 at the introduction end 505 of the sheath 420 aligns with a beginning of the curve of the rounded tip 510 of the catheter 412 such that a transition 512 between the catheter 412 and the sheath 420 is continuous, smooth and substantially edge free. A smooth transition 512 between the catheter 412 and the sheath 420 helps to avoid catching tissue within the patient's nasal cavity 104.

In certain embodiments, the treatment receiving port 436 includes a coupling member 514 for coupling the apparatus 400 to the treatment delivery device. For example, in one embodiment, the coupling member 514 may be a plurality of threads disposed around the circumference of the treatment receiving port 436. The threads of the coupling member 514 engage threads on a syringe or other treatment delivery device to couple the treatment delivery device to the treatment receiving port 436.

Figure 5B depicts a side cutaway view of one embodiment of an apparatus 400 for facilitating intranasal treatment of a patient's sphenopalatine/pterygopalatine recess 118. In the embodiment depicted Figure 5B, the catheter hub 404 is positioned in the extended position 426. The embodiment depicted in Figure 5B is taken along line A-A of Figure 5A and more clearly illustrates one embodiment of the interior surface 502 of the sheath hub 402 and the catheter hub receiving space 504.

In certain embodiments, the sheath hub 402 has an exterior surface 406 that opposes an interior surface 502. The interior surface 502 of the sheath hub 402 defines a catheter hub receiving space 504. A portion 422 of the catheter hub 404 has a reduced diameter and is slideably received within the catheter hub receiving space 504 such that the catheter hub 404 is repositionable in a lengthwise direction along the longitudinal axis 428 (see Figures 4A and 4B) of the sheath hub 402.

In certain embodiments, the apparatus 400 includes an arresting element 516 on either the catheter hub 404 or the sheath hub 402. In the embodiment illustrated in Figure 5B, the arresting element 516 is a flange that is coupled to and extends perpendicularly from an outer surface 518 of the reduced diameter portion 422 of the catheter hub 404.

In one embodiment, the apparatus 400 also includes an engagement element 520 on either the catheter hub 404 or the sheath hub 402. In the embodiment illustrated in Figure 5B, the engagement element 520 is a recess extending longitudinally along the interior surface 502 of the sheath hub 402. The flange of the arresting element 516 is positioned within and travels along the recess of the engagement element 520 when the catheter hub 404 is repositioned along the longitudinal axis 428 of the sheath hub 402. Cooperation between the arresting element 516 and the engagement element 520 allows the catheter hub 404 to be slideably received within the catheter hub receiving space 504 while limiting rotation of the catheter hub 404 with respect to the sheath hub 402. Accordingly, in certain embodiments, the flange of the arresting element 516 is continuously engaged within the recess of the engagement element 520 when the catheter hub 404 is repositioned within along the longitudinal axis 428 of the sheath hub 402. Engagement between the arresting element 516 and the engagement element 520 prevents rotation of the sheath hub 402 with respect to the catheter hub 404.

By limiting rotation of the catheter hub 404 with respect to the sheath hub 402, the physician or other medical professional can determine the orientation of intrinsic curvature 414 of the catheter 412 by the position of the rotational orientation indicator 408a on the catheter hub 404 and the rotational orientation indicator 408b on the sheath hub 402 may be unnecessary.

In certain embodiments, the apparatus 400 also includes a stopping element 522 coupled to either the catheter hub 404 or the sheath hub 402. The stopping element 522 is configured to engage the arresting element 516 to stop the catheter hub 404 from being removed from the catheter hub receiving space 504. In the embodiment illustrated in Figure 5B, the stopping element 522 is a substantially rigid wall that engages the arresting element 516 to stop the catheter hub 404 from being removed from the catheter hub receiving space 504. In certain embodiments, the stopping element 522 also facilitates alignment of the vertex 508 at the introduction end 505 of the sheath 420 with the beginning of the curve of the rounded tip 510 of the catheter 412 such that the transition 512 between the catheter 412 and the sheath 420 is continuous, smooth and substantially edge free when the catheter hub 404 is positioned in the extended position 426.

Of course, one of skill in the art will recognize that in certain embodiments, the position of the arresting element 516, the engagement element 520, and the stopping element 522 may be reversed. For example, in one embodiment, the arresting element 516 may be coupled to the interior surface 502 of the sheath hub 402 and the recess of the engagement element 520 may be positioned in the outer surface 518 of the reduced diameter portion 506 of the catheter hub 404. Similarly, in one embodiment, the stopping element 522 may be coupled to the reduced diameter portion 506 of the catheter hub 404 to restrict the catheter hub 404 from being removed from within the catheter hub receiving space 504 in the sheath hub 402.

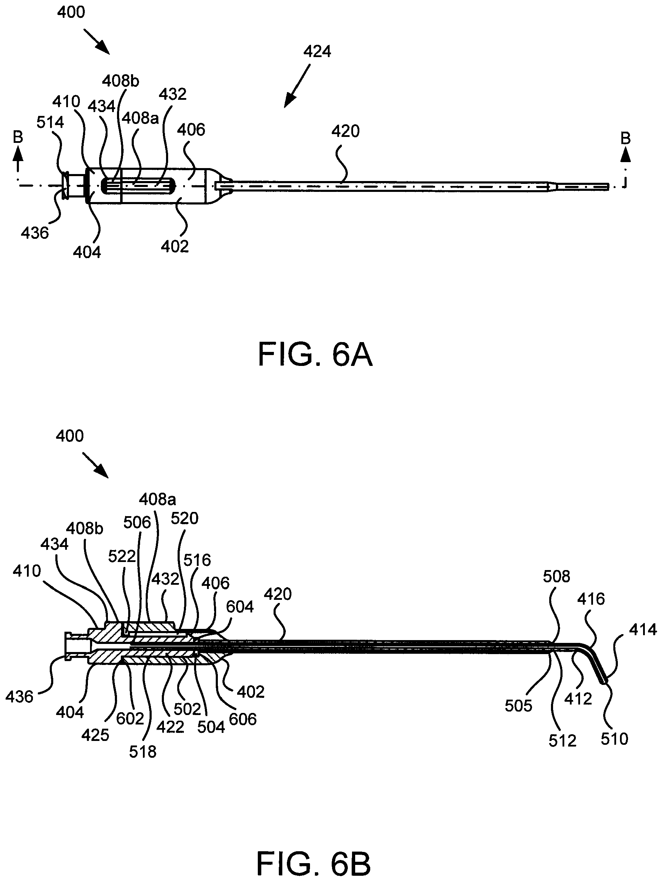

Figure 6A depicts a top view of one embodiment of an apparatus 400 for facilitating intranasal treatment of a patient's sphenopalatine/pterygopalatine recess 118. In the embodiment illustrated in Figure 6A, the catheter hub 404 is positioned in the inserted position 424.

Figure 6B is a side cutaway view of one embodiment of an apparatus for facilitating intranasal treatment of a patient's sphenopalatine/pterygopalatine recess 118. In the embodiment depicted Figure 6B, the catheter hub 404 is positioned in the inserted position 424. The embodiment depicted in Figure 6B is taken along line B-B of Figure 6A.

In one embodiment, when the catheter hub 404 is fully inserted into the catheter hub receiving space 504, the stopping surface 425 of the catheter hub 404 contacts an end 602 of the sheath hub 402 to arrest further insertion of the catheter hub 404 within the catheter hub receiving space 504. In other embodiments, an end 604 of the reduced diameter portion 422 of the catheter hub 404 contacts an interior wall 606 within the catheter hub receiving space 504 to arrest further insertion of the catheter hub 404 within the catheter hub receiving space 504. With the catheter hub 404 fully positioned within the catheter hub receiving space 504 to the point where the stopping surface 425 contacts the sheath hub 402, the catheter hub 404 may be considered to be positioned in the fully inserted position 424.

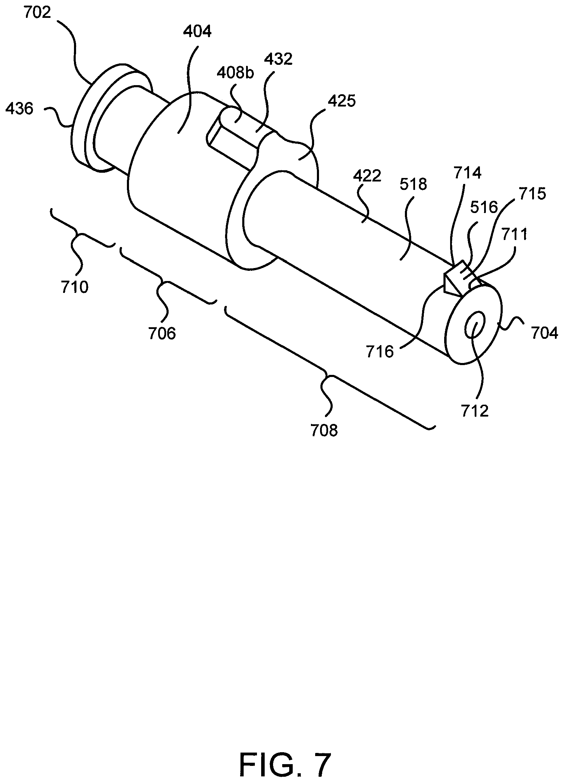

Figure 7 depicts a perspective view of one embodiment of a catheter hub 404 according to the present disclosure. In the embodiment illustrated in Figure 7, the sheath hub 402 and the catheter 412 have been omitted to better illustrate the catheter hub 404.

In certain embodiments, the catheter hub 404 includes a treatment receiving end 702 disposed opposite a treatment delivery end 704. The catheter hub 404 includes a manipulation portion 706, an insertion portion 708, and a coupling portion 710. In the depicted embodiment, the manipulation portion 706, the insertion portion 708, and the coupling portion 710 are substantially cylindrical. In other embodiments, the manipulation portion 706, the insertion portion 708, and/or the coupling portion 710 may have triangular profiles, square profiles, rectangular profiles, octagonal profiles, oblong profile, or a profile having any other geometric shape.

The insertion portion 708 of the catheter hub 404 includes the reduced diameter portion 422 of the catheter hub 404. The insertion portion 708 begins at the stopping surface 425 of the catheter hub 404 and extends to the treatment delivery end 704 of the catheter hub 404.

The coupling portion 710 of the catheter hub 404 is positioned opposite the insertion portion 708 and includes the treatment receiving port 436. Anesthetics, medications, an electrical current, or any other treatment is received at the treatment receiving port 436 and delivered to the treatment delivery end 704 through a lumen 712 disposed through the catheter hub 404 from the treatment receiving end 702 to the treatment delivery end 704. When a catheter 412 is positioned in the lumen 712, the catheter receives the treatment and delivers it to a desired area such as the patient's sphenopalatine/pterygopalatine recess 118.

The manipulation portion 706 of the catheter hub 404 has an increased diameter with respect to the insertion portion 708 and is positioned between the coupling portion 710 and the insertion portion 708. The increased diameter of the manipulation portion 706 of the catheter hub 404 facilitates manipulation of the catheter hub 404 by the physician or other medical professional.

The arresting element 516 is coupled to and extends substantially perpendicularly from the outer surface 518 of the insertion portion 708 of the catheter hub 404. The arresting element 516, in one embodiment, includes a sloped surface 715. For example, in one embodiment an end 711 of the arresting element 516 adjacent the treatment delivery end 704 of the catheter hub 404 extends from the outer surface 518 of the insertion portion 708 of the catheter hub 404 a distance that is substantially less than an end 714 of the arresting element 516 closer to the treatment receiving end 702 of the catheter hub 404. The sloped surface 715 of the arresting element 516 allows the flange of the arresting element 516 to be inserted past the stopping element 522 and into the recess of the engagement element 520. A rear surface 716 of the arresting element 516 extends substantially perpendicularly from the outer surface 518 of the reduced diameter portion 422 of the catheter hub 404. When the catheter hub 404 is withdrawn from within the catheter hub receiving space 504, the rear surface 716 of the arresting element 516 engages the stopping element 522 to stop the catheter hub 404 from being removed from within the catheter hub receiving space 504. In certain embodiments, the interaction between the rear surface of the arresting element 516 and the stopping element 522 positions the catheter 412 within the sheath 420 such that the vertex 508 at the introduction end 505 of the sheath 420 aligns with a beginning of the curve of the rounded tip 510 of the catheter 412. In this position the transition 512 between the catheter 412 and the sheath 420 is continuous, smooth and substantially edge free.

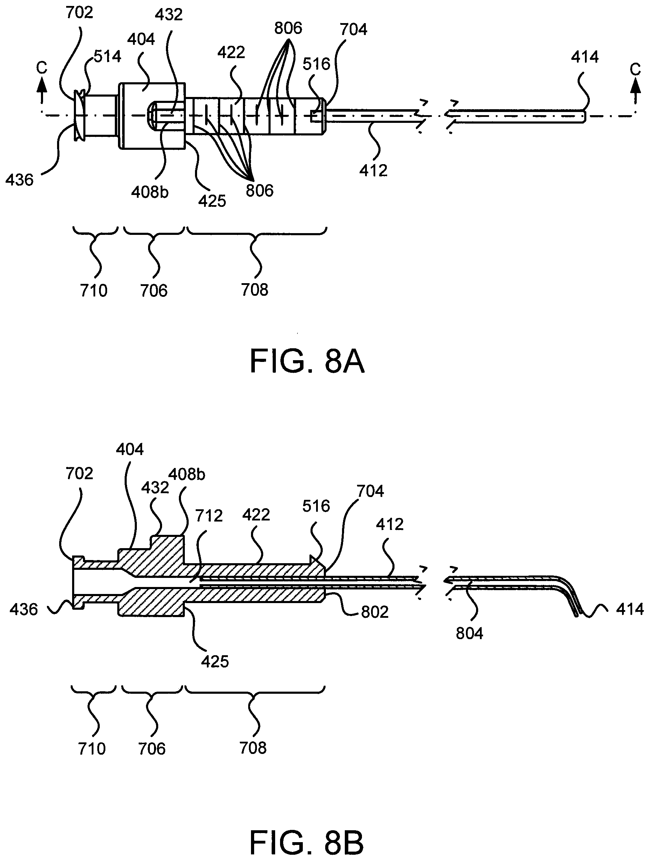

Figure 8A depicts a side view of one embodiment of a catheter hub 404 and a catheter 412 according to the present disclosure. In the embodiment illustrated in Figure 8A, the threads of the coupling member 514 are more clearly illustrated. In other embodiments, the coupling member 514 may be a snap fit attachment, a rim for chemically adhering a treatment delivery device, or any other means for attaching or affixing the treatment delivery device to the catheter hub 404.

In certain embodiments, the catheter hub 404 includes a plurality of depth indicators 806 disposed along the insertion portion 708 of the catheter hub 404 at regular intervals. In the embodiment illustrated in Figure 8A, the depth indicators 806 are lines positioned around the circumference of the insertion portion 708 of the catheter hub 404. In other embodiments, the depth indicators 806 may include other shapes, such as dots, squares, circles, triangles, or any other visual indicator. In one embodiment, the depth indicators 806 may also include a numerical indication of a depth of the catheter hub 404 within the catheter hub receiving space 504.

In performing an initial nerve block, with the catheter hub 404 positioned in the extended position 426, the physician or other medical professional advances the sheath 420 and the catheter 412 into the patient's nasal cavity 104. Once the insertion end 414 of the catheter has passed the anterior ridge 302 of the middle sinus turbinate 114, the physician or other medical professional advances the catheter hub 404 deeper within the catheter hub receiving space 504 causing the insertion end 414 of the catheter 412 to advance past the introduction end 505 of the sheath 420. Once the insertion end 414 of the catheter 412 is advanced to a position where the sheath 420 no longer contains the intrinsic curvature 416 of the catheter 412, the catheter 412 bends. Because the physician or other medical professional knows the direction in which the catheter 412 is bent by the orientation of the rotational orientation indicator 408, the physician or other medical professional can direct the insertion end 414 of the catheter 412 into the sphenopalatine/pterygopalatine recess 118 to deliver a nerve block or other treatment to the sphenopalatine/pterygopalatine ganglia 120.

As will be evident to one of skill in the art, the anatomy of a patient's nasal cavity 104 varies from individual to individual. Accordingly, certain patient's will have a deeper sphenopalatine/pterygopalatine recess 118 than others. The depth indicators 806 on the insertion portion 708 of the catheter hub 404 allow a physician or other medical professional to determine a depth of the insertion end 414 of the catheter 412 when the insertion end 414 of the catheter 412 is positioned within the patient's sphenopalatine/pterygopalatine recess 118. In one embodiment, during an initial treatment of a particular patient's sphenopalatine/pterygopalatine ganglia 120 the physician or other medical professional may record a depth of the patient's sphenopalatine/pterygopalatine recess 118. In subsequent treatments of the patient's sphenopalatine/pterygopalatine ganglia 120, the physician or other medical professional can use the recorded depth as a guide.

Figure 8B depicts a cutaway view of one embodiment of a catheter hub 404 according to the present disclosure. The embodiment depicted in Figure 8B is taken along line C-C of Figure 8A.

In certain embodiments, the catheter 412 is positioned within the lumen 712 in the catheter hub 404 and extends at least partially into the lumen 712. In other embodiments, the catheter 412 may be affixed to the end surface 802 at the treatment delivery end 704 of the catheter 412. In either embodiment, the catheter 412 is communicable in fluid communication with the lumen 712 to deliver medication, an anesthetic, or other chemical to the insertion end 414 of the catheter 412 where it can be dispensed to the patient.

In other embodiments, a electrical conduit, such as a wire, is positioned through the lumen 712 in the catheter 412 and through a lumen 804 in the catheter. In such an embodiment, the electrical conduit may be coupled to an electrical source to deliver an electrical current to an electrode positioned at the insertion end 414 of the catheter 412. The electrode is configured to deliver the electrical current to the patient.

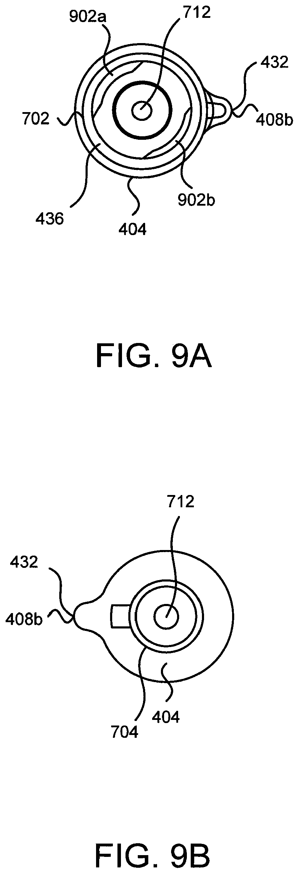

Figure 9A is an end view depicting one embodiment of a catheter hub 404 according to the present disclosure. The embodiment illustrated in Figure 9A is taken in the direction of the treatment receiving end 702 of the catheter hub 404.

In certain embodiments, the catheter hub 404 includes one or more coupling flanges 902a and 902b positioned on an inner circumference 904 of the treatment receiving port 436. In such an embodiment, the coupling flanges 902 are configured to couple a syringe or other treatment delivery device to the catheter hub.

Figure 9B is an end view depicting one embodiment of a catheter hub 404 according to the present disclosure. The embodiment illustrated in Figure 9A is taken in the direction of the treatment delivery end 704 of the catheter hub 404.

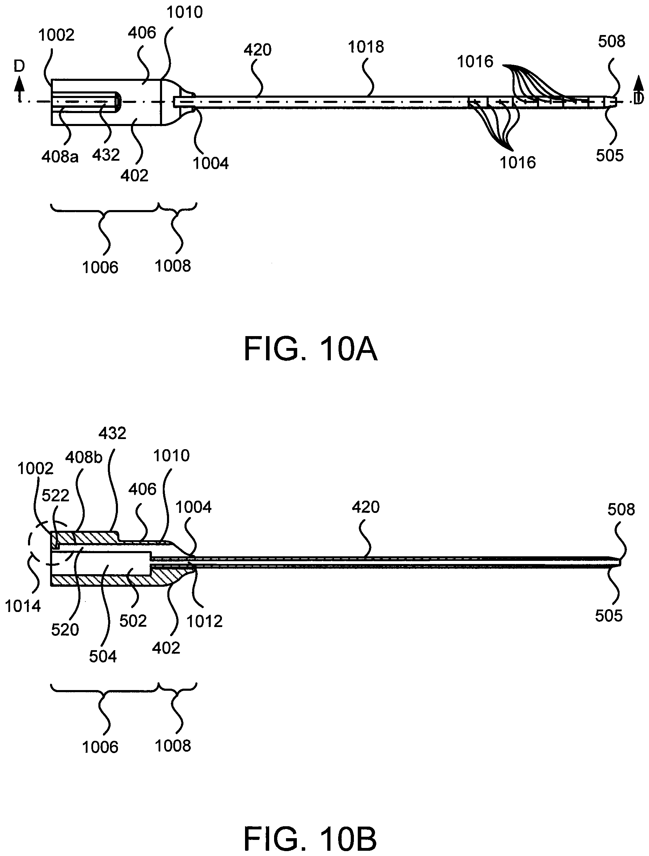

Figure 10A depicts a top view of one embodiment of the sheath hub 402 coupled to a sheath 420 according to the present disclosure. The sheath hub 402, in certain embodiments, includes a catheter hub receiving end 1002 and a sheath receiving end 1004. In the embodiment illustrated in Figure 10A, the sheath 420 is coupled to the sheath receiving end 1004 of the sheath hub 402.

In certain embodiments, the sheath hub 406 includes a substantially cylindrical portion 1006 and a tapered portion 1008. Disposed within the cylindrical portion 1006 is the catheter hub receiving space 504. In certain embodiments, the catheter hub receiving space 504 is also a substantially cylindrical void in the sheath hub 406. In such embodiments, the insertion portion 708 of the catheter hub 404 is also cylindrical such that the insertion portion 708 of the catheter hub 404 may be received within the catheter hub receiving space. In other embodiments, the shape of the insertion portion 708 of the catheter hub 404 and the shape of the void of the catheter hub receiving space 504 may be any other geometric shape while the outer surface 406 of the sheath hub 402 remains cylindrical. Of course, in one embodiment, the opposite may be true. That is, in certain embodiments, the outer surface 406 of the sheath hub 402 may be a shape other than cylindrical while the shape of the insertion portion 708 of the catheter hub 404 and the shape of the void of the catheter hub receiving space 504 are cylindrical.

The tapered portion 1008 of the sheath hub 402 extends from the cylindrical portion 1006 to the sheath receiving end 1004 of the sheath hub 402. The diameter of the sheath hub 402 at the sheath receiving end 1004 is substantially smaller than the diameter of the sheath hub 402 at the interface 1010 between the cylindrical portion 1006 and the tapered portion 1008 such that the tappered portion 1008 of the sheath hub 402 is sloped towards the tappered portion 1008 of the sheath hub 402.

In certain embodiments, the sheath 420 may include a plurality of depth indicators 1016 disposed along at least a portion of an exterior surface 1018 of the sheath 420 at regular intervals. In the embodiment illustrated in Figure 10A, the depth indicators 1016 are lines positioned on the exterior surface 1018 of the sheath 420. In other embodiments, the depth indicators 1016 may include other shapes, such as dots, squares, circles, triangles, or any other visual indicator. In one embodiment, the depth indicators 1016 may also include a numerical indication of a depth of the sheath 420 when the sheath 420 is positioned within a patient's nasal cavity 104.

As discussed above, the anatomy of a patient's nasal cavity 104 varies from individual to individual. Accordingly, a depth of the anterior ridge 302 of the middle sinus turbinate 114 varies from patient to patient. During an initial treatment, a physician or other medical professional may use the depth indicators 1016 on the sheath 420 to record a depth of the anterior ridge 302 of the middle sinus turbinate 114 for a particular patient. In certain embodiments, the physician or other medical professional may also record a depth of the inferior surface 304 of the nasal bone 122 for the patient. For subsequent treatments, the physician or other medical professional can reference the recorded depths to avoid contacting or damaging the delicate tissues within the patient's nasal cavity 104.

In one embodiment, for the initial treatment, the physician or other medical professional may refer to a table (not shown) that lists an average depth anterior ridge 302 of the middle sinus turbinate 114 and an average depth of the inferior surface 304 of the nasal bone 122 for a patient according to certain characteristics of the patient. For example, in one embodiment, the table may list the average depth anterior ridge 302 of the middle sinus turbinate 114 and the average depth of the inferior surface 304 of the nasal bone 122 for a given age group. The table may also list an average depth of the sphenopalatine/pterygopalatine recess 118 for a given age group. In certain embodiments, the table may be further broken down into gender classifications. In another embodiment, the table may list the average depths according to measurements taken on the patient's external nasal anatomy.

Figure 10B depicts a cutaway view of one embodiment of a sheath hub 402 coupled to a sheath 420 according to the present disclosure. The embodiment depicted in Figure 10B is taken along line D-D of Figure 10A.

In certain embodiments, the tapered portion includes a cavity 1012 that extends through the tapered portion from the catheter hub receiving space 504 in the sheath hub 402 to the sheath receiving end 1004 of the sheath hub 402. The sheath 420 is received within the cavity 1012 to couple the sheath to the sheath hub 402.

In the embodiment illustrated in Figure 10B, the engagement element 520 is more clearly shown as a recess extending longitudinally along the interior surface 502 of the sheath hub 402. In certain embodiments, the recess of the engagement element 520 is only positioned within the cylindrical portion 1006 of the sheath hub 402. In such an embodiment, the recess of the engagement element 520 may extend from the stopping element 522 to the interface 1010 between the cylindrical portion 1006 and the tapered portion 1008. In other embodiments, such as the embodiment illustrated in Figure 10B, the recess of the engagement element 520 may extend through the tapered portion 1008 of the sheath hub 402.

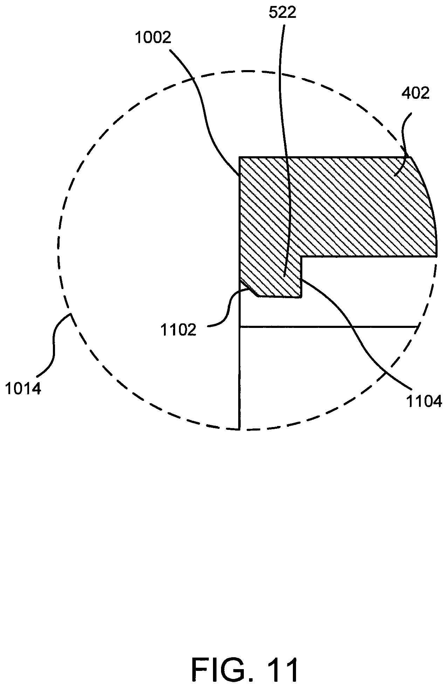

Figure 11 depicts an enlarged view of area 1014 of the sheath hub 402 which includes the stopping element 522 according to one embodiment of the present disclosure. In certain embodiments, the stopping element 522 includes a sloped surface 1102 disposed adjacent the catheter hub receiving end 1002 of the sheath hub 402. The sloped surface 1102 of the stopping element 522 may be engaged by the sloped surface 715 of the flange of the arresting element 516 to facilitate easy insertion of the insertion portion 708 of the catheter hub 404 into the catheter hub receiving space 504. In certain embodiments, the stopping element 522, the flange of the arresting element 516, or both may be made of a pliable or semi-pliable material to facilitate easy insertion of the insertion portion 708 of the catheter hub 404 into the catheter hub receiving space 504. In other embodiments, the entire catheter hub 402, the entire sheath hub 402, or both may be made of a pliable or semi-pliable material.

A stopping surface 1104 of the stopping element 522 extends substantially perpendicularly from the interior surface 502 of the sheath hub 402. When the catheter hub 404 is withdrawn from within the catheter hub receiving space 504 to the extended position 426, the stopping surface 1104 of the stopping element 522 engages the rear surface 716 of the arresting element 516 to stop further withdrawal of the catheter hub 404 from within the catheter hub receiving space 504.

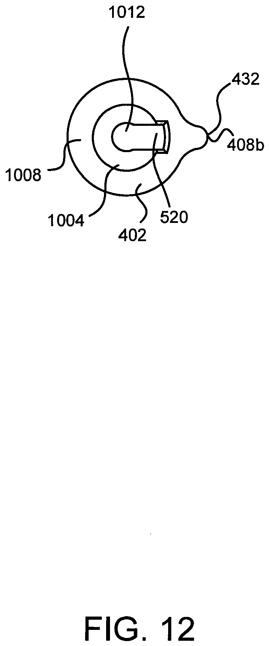

Figure 12 depicts an end view of one embodiment of the sheath hub 402 according to the present disclosure. The embodiment illustrated in Figure 12 is taken in the direction of the sheath receiving end 1004 of the sheath hub 402, the sheath 420 has been removed for clarity.

In the embodiment illustrated in Figure 12, the recess of the engagement element 520 extends through the tapered portion 1008 of the sheath hub 402 all the way to the sheath receiving end 1004. In certain embodiments, the recess of the engagement element 520 is aligned in the same rotational orientation with respect to a longitudinal axis of the sheath hub 402 as the rotational orientation indicator 408b. In other embodiments, the recess of the engagement element 520 may be offset from the rotational orientation indicator 408b.

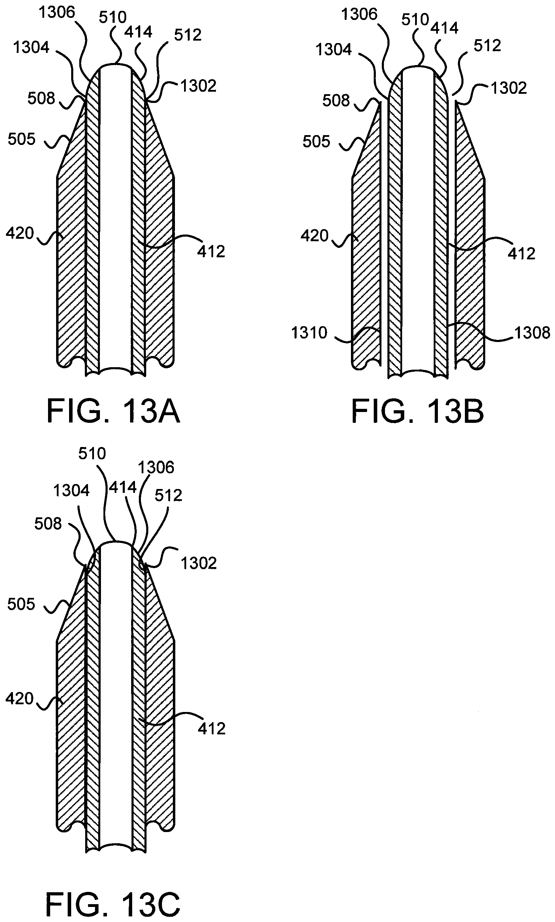

Figure 13A illustrates a cutaway view of one embodiment of an introduction end 505 of the sheath 420 and an insertion end 414 of the catheter 412. In the embodiment illustrated in Figure 13A, the introduction end 505 of the sheath 420 and the insertion end 414 of the catheter 412 are positioned in the position that the introduction end 505 of the sheath 420 and the insertion end 414 of the catheter 412 are placed when the catheter hub 404 (not shown) is positioned in the extended position 426.

In certain embodiments, the tip 510 of the insertion end 414 of the catheter 412 is curved 1306 such that the tip 510 is rounded. An outermost edge 1302 at the introduction end 505 of the sheath 420 is sloped to a vertex 508. In one embodiment, when the catheter hub 404 is position in the extended position 426, the vertex 508 at the introduction end 505 of the sheath 420 is aligned with a beginning 1304 of the curve 1306 of the rounded tip 510 of the catheter 412. In such an embodiment, the transition 512 between the catheter 412 and the sheath 420 is continuous, smooth and substantially edge free. In one embodiment, the fit between the catheter 412 and the sheath 420 is tight. That is, in one embodiment, there is substantially no gap between the catheter 412 and the sheath 420. In one embodiment, a lack of a gap between the catheter 412 and the sheath 420 reduces the likelihood that the vertex 508 at the introduction end 505 of the sheath 420 will snag or otherwise damage the delicate tissues within the nasal cavity 104 of the patient.

Figure 13B illustrates a cutaway view of one embodiment of an introduction end 505 of the sheath 420 and an insertion end 414 of the catheter 412. In the embodiment illustrated in Figure 13B, the outer diameter 1308 of the catheter 412 is substantially smaller than the inner diameter 1310 of the sheath 1420. In such an embodiment, the transition 512 between the catheter 412 and the sheath 420 is not smooth and the vertex 508 on the introduction end 505 of the sheath 420 may form a sharp edge that may snag or otherwise damage the delicate tissues within the nasal cavity 104 of the patient. Accordingly, in certain embodiments, the catheter 412 and the sheath 420 may be designed such that the fit between the catheter 412 and the sheath 420 is tight to avoid sharp edges.

Figure 13C illustrates a cutaway view of another embodiment of an introduction end 505 of the sheath 420 and an insertion end 414 of the catheter 412. In the embodiment illustrated in Figure 13C, the catheter 412 is positioned within the sheath 420 at a position that causes the vertex 508 of the sheath 420 to extend beyond the beginning 1304 of the curve 1306 at the insertion end 414 of the catheter 412. In such an embodiment, the transition 512 between the catheter 412 and the sheath 420 is not smooth and the vertex 508 on the introduction end 505 of the sheath 420 may form a sharp edge that may snag or otherwise damage the delicate tissues within the nasal cavity 104 of the patient. Accordingly, in one embodiment, the stopping element 522 stops the withdrawal of the catheter hub 404 from within the catheter hub receiving space 504 at a position that aligns the vertex 508 on the introduction end 505 of the sheath 420 with the beginning 1304 of the curve 1306 at the insertion end 414 of the catheter 412.

Unexpectedly, advanced prototype catheters have been effectively used, with the informed consent of patients, to alleviate headache pain. Results of those procedures have been recorded and are encouraging. After 74 procedures, only four patients rated the tolerability of the procedure as either "poor" or "fair." Of the remaining patients, 24 rated tolerability as "good" and 46 rated it "excellent." This tolerability improved when patients were pretreated with intranasal anesthetic, as summarized in Table 1 below.

Of the 74 procedures recorded, no adverse events required intervention. On five occasions the nasal mucous was noted to be slightly blood-tinged, but no frank epistaxis occurred. Two procedures resulted in worsened headache, but both patients were back to their baseline headache the following day without further incident. Except for those two patients, all responded that they would have the procedure again if necessary.

On the day that SPGB was performed, 58% of patients left the office with a complete resolution of their headache, while 74% experienced significant clinical improvement as assessed by the visual analog scale (VAS). Though some patients have been lost to follow-up, significant improvement in headache severity persisted for one third of patients at one month. These results defy expected norms both in terms of clinical outcomes and predicted efficacy both of the device and the procedure. Likewise, unexpected is ostensive patient acceptance and tolerability of the procedure. Given the nature of the procedural and patient satisfaction outcomes, performance of this procedure by other than the most highly skilled, experienced and practiced surgeons, it is respectfully proposed, constitutes progress in science and the useful arts as based upon an unexpected series of results, according to the person of normal skill in the art, in this case, it is respectfully submitted, a neurologist or neurosurgeon.

**Table 1:**

| **Reduction in VAS** | **At Discharge (N=74)** |
|---|---|
| 80-100% | 43 (58%) |
| 50-79% | 12 (16%) |
| 20-49% | 3 (4%) |
| <19% | 16 (23%) |

### EXAMPLES

BC sustained a head injury in a snowmobile accident nearly a decade ago and could not remember a day without a headache since. Therapy, standard medications, even narcotics failed to give him relief. Every day ended with an 8/10 headache. He experienced 100% resolution of his headache after SPGB and has remained headache free for more than a year.

AD, a highway patrolman, suffered 8/10 headaches virtually every day for years. He remained headache free for two months after SPGB. Remembering in those two months how good life could be without headaches, he tearfully requested a repeat procedure when his headaches returned. He now has a brief, painless, bi-monthly procedure and lives headache free.

Likewise, certain types of headaches are a consequence of nerve dysfunction, disrupting the dysfunctional circuits can act as a reset, allowing normal nerve function to return. For this reason, the benefit of a SPGB may last for an indefinite period of time, far beyond the effect of the local anesthetic. Accordingly, it has been learned that many patients experience an increased benefit when the procedure is repeated.

Prior teachings fail to suggest such results. For example, a randomized, double-blind, controlled trial published in the Journal of the American Medical Association in 1996 described rapid relief of migraine headaches in 55% of patients when they dripped topical lidocaine into the nostrils of headache sufferers. Of those who responded, 42% experienced relapse headaches, usually within the first hour. Of note, they excluded patients whose "headache had lasted more than 3 days or if the frequency of severe headaches was more than once per week."

Our patients suffered from chronic daily headaches, many with the very types of headaches excluded by this study. Because there is good scientific theory behind SPGB for several types of cephalgia, and because the potential adverse effects of the procedure are so few and so mild, we took the approach of being much more inclusive in our use of SPGB and have experienced better than expected results.

A retrospective chart review at Thomas Jefferson University in 2006 looked at a refractory chronic headache population more similar to ours, including 41 patients with transformed migraines, 12 with daily persistent headaches and 15 with "other headache diagnoses." They reported; "25.4% had complete a response, 57.1% had a partial response, 3.2% worsened, and 14.3% had no change." Their lidocaine regimen, however, was *IV lidocaine in a cardiac monitored unit over 2-15 days.*

It is respectfully proposed that the SphenoCath™ brand of medical device has the potential to eclipse most, if not all, published study results, simply because it more precisely and consistently delivers medication to the desired location. Direct fluoroscopy has documented the ability of the SphenoCath™ brand of medical device to deliver an infusion to the target. Furthermore, the SphenoCath™ brand of medical device extends the opportunity for SPGB from a small contingent of pain specialists, to the office practitioner, exponentially increasing patient access to the procedure.

In certain embodiments, the vertex 508 at the introduction end 505 of the sheath 420 is shielded from the delicate tissues within the nasal cavity 104 of the patient by the rounded side wall 1314 of the bulbous tip area 1310. In other embodiments, an outer diameter 1316 of the sheath 420 may be substantially smaller than an outermost diameter 1318 of the bulbous tip area 1310 such that the bulbous tip area shields the introduction end 505 of the sheath 420 from potential snags or other damage to the delicate tissues within the nasal cavity 104 of the patient. In such an embodiment, the introduction end 505 may be squared off or rounded rather than sloped as illustrated in the depicted embodiment.

Those skilled in the art will readily understand use of the present invention for indications, at least one of, Head and neck cancer, Complex regional pain syndrome, Reflex Sympathetic Dystrophy, Vasomotor rhinitis, Pre- and postoperative anesthesia in oral and maxillofacial surgery, Cluster Headache, Cephalgia, Cervical Spasm, Disc Disease or Herniation, Any Level, Lumbago, Lumbosacral Spasm, Pyriformis Spasm Syndrome, Spastic (Spasmodic) Torticollis, SPG Neuralgia (Sluder's Syndrome), Trigeminal Neuralgia (Tic Douleureux), Post-Herpetic Neuralgia, Autonomic Neuralgia, Atypical Facial Pain, Bulging Disc, Lumbosacral Disc, Causalgia, Reflex Sympathetic Dystrophy (RSD), Cervical Spondylosis, Migraine Headache, Sinus Headache, Cerebrospinal Fluid (CSF) Leak Headache, Chronic Sinusitis, Tension Headache, Myofascial Pain Syndrome, Pinched Nerve, Sciatica, Slipped Disc, Sinus Pain, Temporomandibular Joint Syndrome (TMJ), Whiplash, Allergic Rhinitis, Vasomotor Rhinitis, Asthma, Bell's Palsy (Facial Nerve Palsy), Bone Pain, Cancer Pain, Bronchospasm, Chronic Bronchitis, Dysmenorrhea, Endometriosis, Fibromyalgia, Multiple Sclerosis with Spasm, Peripheral Neuropathy (Neuropathic Pain), Raynaud's Phenomenon, Rheumatoid Arthritis (Flare), Shingles (Herpes Zoster), Spinal Stenosis, Chronic Fatigue Syndrome, Chronic Hiccup, Diabetic Neuropathy, Hyperhydrosis, Intracranial Hypotension Headache, Phantom Limb/Tooth Pain, Paresthesisas, Repetitive Stress Injury, and Tinnitus (Low Frequency).

The described embodiments are to be considered in all respects only as illustrative and not restrictive. The scope of the subject matter is, therefore, indicated by the appended claims rather than by the foregoing description.

While the method and apparatus have been described in terms of what are presently considered to be the most practical, it is to be understood that the disclosure need not be limited to the disclosed implementations. The present disclosure also includes any and all implementations of the following claims.

It should also be understood that a variety of changes may be made without departing from the essence of the disclosure. Such changes are also implicitly included in the description. They still fall within the scope of this disclosure.

Further, each of the various elements of the disclosure and claims may also be achieved in a variety of manners. This disclosure should be understood to encompass each such variation, be it a variation of an implementation of any apparatus implementations, a method or process implementations, or even merely a variation of any element of these.

It should be understood that all actions may be expressed as a means for taking that action or as an element which causes that action.

Similarly, each physical element, disclosed, should be understood to encompass a disclosure of the action which that physical element facilitates.

Further, the use of the transitional phrase "comprising" is used to maintain the "open-end" claims herein, according to traditional claim interpretation. Thus, unless the context requires otherwise, it should be understood that the term "comprise" or variations such as "comprises" or "comprising", are intended to imply the inclusion of a stated element or step or group of elements or steps but not the exclusion of any other element or step or group of elements or steps.

## Claims

1. An apparatus (400) facilitating intranasal treatment of a patient's sphenopalatine/pterygopalatine recess, the apparatus (400) comprising, in combination:
a sheath hub (402) having an exterior surface (406) opposing an interior surface (502), wherein the interior surface (502) defines a catheter hub receiving space (504);
a catheter hub (404) slideably received within the catheter hub receiving space (504), wherein the catheter hub (404) is positionable along a longitudinal axis of the sheath hub (402);
a sheath (420) coupled to the sheath hub (402) and a catheter (412) coupled to the catheter hub (404) and received within the sheath (420), wherein the catheter (412) includes an insertion end (414) and a coupling end (506), the insertion end (414) having an intrinsic curvature (416) with respect to a longitudinal axis of the catheter (412), wherein the catheter hub (404) is positionable between an inserted position (424) and an extended position (426) and the sheath (420) straightens the intrinsic curvature (416) of the catheter (412), when the catheter hub (404) is positioned in the extended position (426),
an arresting element (516) on one of the catheter hub (404) and the sheath hub (402); and
an engagement element (520) on the other of the catheter hub (404) and the sheath hub (402), wherein the arresting element (516) continuously engages the engagement element (520) when the catheter hub (404) is repositioned along the longitudinal axis of the sheath hub (402) and wherein engagement between the arresting element (516) and the engagement element (520) prevents rotation of the sheath hub (402) with respect to the catheter hub (404),
**characterized in that**
the catheter hub (404) comprises a manipulation portion (706), an insertion portion (708) and a coupling portion (710), wherein the manipulation portion (706) has an increased diameter with respect to the insertion portion (708) and is positioned between the coupling portion (710) and the insertion portion (708), wherein the insertion portion (708) begins at a stopping surface (425) of the catheter hub (404) and extends to a treatment delivery end (704), and wherein the stopping surface (425) of the catheter hub (404) contacts the sheath hub (402) to arrest further insertion of the catheter hub (404) within the catheter hub receiving space (504) when the insertion portion (708) of the catheter hub (404) is fully inserted into the catheter hub receiving space (504).

2. The apparatus (400) of claim 1, further comprising a rotational orientation indicator (408), the rotational orientation indicator (408) identifying a rotational orientation of the intrinsic curvature (416) of the catheter (412).

3. The apparatus (400) of claim 2, wherein the rotational orientation indicator (408) comprises a raised ridge (432) extending longitudinally along at least one of the sheath hub (412) and the catheter hub (404).

4. The apparatus (400) of claim 1, wherein the catheter hub (404) includes at least one depth indicator (806) that indicates a position of the insertion end (414) of the catheter (412) relative to an introduction end (505) of the sheath (420).

5. The apparatus (400) of claim 1, wherein the insertion end (414) having a tip (510) that is curved such that the tip (510) is rounded, the sheath comprising an introduction end (505) having an outermost edge (1302) that is sloped to a vertex (508) and wherein the vertex (508) aligns with a beginning (1304) of the curve (1306) of the rounded tip (510) of the catheter (412) when the catheter hub (404) is positioned in the extended position (426).

6. The apparatus (400) of claim 5, wherein a transition between the vertex (508) and the beginning (1304) of the curve (1306) of the rounded tip (510) of the catheter (412) is continuous when the catheter hub (404) is positioned in the extended position (426).

7. The apparatus (400) of claim 5, wherein the tip (510) of the insertion end of the catheter (412) is bulbous.

8. The apparatus (400) of claim 1, wherein the arresting element (516) comprises a flange coupled to and extending perpendicularly from one of an outer surface (18) of the catheter hub (404) and an interior surface (502) of the sheath hub (402), wherein the engagement element (520) comprises a recess extending longitudinally along the other of the interior surface (502) of the sheath hub (402) and the outer surface (18) of the catheter hub (404), wherein the flange is positioned within and travels along the recess when the catheter hub (404) is repositioned along the longitudinal axis of the sheath hub (402).

9. The apparatus (400) of claim 1, further comprising a stopping element (522) coupled to one of the catheter hub (404) and the sheath hub (402), wherein the catheter hub (404) is positionable along the longitudinal axis of the sheath hub (402) between an inserted position (424) and an extended position (426), wherein the stopping element (522) is configured to engage the arresting element (516) to stop the catheter hub (404) from being removed from the catheter hub receiving space (504).

## Patentansprüche

1. Vorrichtung (400) zum Erleichtern von intranasaler Behandlung der sphenopalatinen/pterygopalatinen Vertiefung eines Patienten, wobei die Vorrichtung (400) in Kombination umfasst:
einen Mantelansatz (402) mit einer Außenoberfläche (406), die einer Innenoberfläche (502) gegenüberliegt, wobei die Innenoberfläche (502) einen Katheteransatz-Aufnahmeraum (504) definiert;
einen Katheteransatz (404), der verschiebbar in dem Katheteransatz-Aufnahmeraum (504) aufgenommen ist, wobei der Katheteransatz (404) entlang der Längsachse des Mantelansatzes (402) positionierbar ist;
einen Mantel (420), der an den Mantelansatz (402) und einen Katheter (412) gekoppelt ist, der an den Katheteransatz (404) gekoppelt ist und in den Mantel (420) aufgenommen ist, wobei der Katheter (412) ein Einführende (414) und ein Kopplungsende (506) aufweist, wobei das Einführende (414) eine inhärente Krümmung (416) bezogen auf die Längsachse des Katheters (412) aufweist, wobei der Katheteransatz (404) zwischen einer eingeführten Stellung (424) und einer ausgefahrenen Stellung (426) positionierbar ist und der Mantel (420) die inhärente Krümmung (416) des Katheters (412) geraderichtet, wenn der Katheteransatz (404) in der ausgefahrenen Stellung (426) positioniert ist,
ein Feststellelement (516) an einem von dem Katheteransatz (404) und dem Mantelansatz (402); und
ein Eingriffselement (520) an dem anderen von dem Katheteransatz (404) und dem Mantelansatz (402), wobei das Feststellelement (516) ständig mit dem Eingriffselement (520) in Eingriff steht, wenn der Katheteransatz (404) entlang der Längsachse des Mantelansatzes (402) repositioniert wird, und wobei der Eingriff zwischen dem Feststellelement (516) und dem Eingriffselement (520) eine Drehung des Mantelansatzes (402) relativ zu dem Katheteransatz (404) verhindert,
**dadurch gekennzeichnet, dass**
der Katheteransatz (404) einen Manipulationsteil (706), einen Einführteil (708) und einen Kopplungsteil (710) umfasst, wobei der Manipulationsteil (706) einen größeren Durchmesser als der Einführteil (708) aufweist und zwischen dem Kopplungsteil (710) und dem Einführteil (708) angeordnet ist, wobei der Einführteil (708) bei einer Anschlagoberfläche (425) des Katheteransatzes (404) beginnt und zu einem Behandlungs-Abgabeende (704) verläuft, und wobei die Anschlagoberfläche (425) des Katheteransatzes (404) in Kontakt mit dem Mantelansatz (402) kommt, um weiteres Einführen des Katheteransatzes (404) in den Katheteransatz-Aufnahmeraum (504) zu verhindern, wenn der Einführteil (708) des Katheteransatzes (404) vollständig in den Katheteransatz-Aufnahmeraum (504) eingeführt ist.

2. Vorrichtung (400) gemäß Anspruch 1, ferner umfassend einen Drehorientierungsanzeiger (408), wobei der Drehorientierungsanzeiger (408) die Drehorientierung der inhärenten Krümmung (416) des Katheters (412) anzeigt.

3. Vorrichtung (400) gemäß Anspruch 2, wobei der Drehorientierungsanzeiger (408) eine erhöhte Rippe (432) umfasst, die längslaufend entlang wenigstens einer von dem Mantelansatz (412) und dem Katheteransatz (404) verläuft.

4. Vorrichtung (400) gemäß Anspruch 1, wobei der Katheteransatz (404) wenigstens einen Tiefenanzeiger (806) aufweist, der die Stellung des Einführendes (414) des Katheters (412) relativ zu dem Einführende (505) des Mantels (420) anzeigt.

5. Vorrichtung (400) gemäß Anspruch 1, wobei das Einführende (414) eine Spitze (510) aufweist, die gekrümmt ist, so dass die Spitze (510) abgerundet ist, wobei der Mantel ein Einführende (505) umfasst, das einen äußersten Rand (1302) aufweist, der zu einem Scheitel (508) abgeschrägt ist, wobei der Scheitel (508) mit dem Beginn (1304) der Krümmung (1306) der abgerundeten Spitze (510) des Katheters (412) fluchtet, wenn der Katheteransatz (404) in der ausgefahrenen Stellung (426) positioniert ist.

6. Vorrichtung (400) gemäß Anspruch 5, wobei der Übergang zwischen dem Scheitel (508) und dem Beginn (1304) der Krümmung (1306) der abgerundeten Spitze (510) des Katheters (412) kontinuierlich ist, wenn der Katheteransatz (404) in der ausgefahrenen Stellung (426) positioniert ist.

7. Vorrichtung (400) gemäß Anspruch 5, wobei die Spitze (510) des Einführendes des Katheters (412) bauchig ist.

8. Vorrichtung (400) gemäß Anspruch 1, wobei das Feststellelement (516) einen Flansch umfasst, der an eines von einer Außenoberfläche (18) des Katheteransatzes (404) und einer Innenoberfläche (502) des Mantelansatzes (402) gekoppelt ist und senkrecht davon vorragt, wobei das Eingriffselement (520) eine Vertiefung aufweist, die längslaufend entlang dem anderen von der Innenoberfläche (502) des Mantelansatzes (402) und der Außenoberfläche (18) des Katheteransatzes (404) verläuft, wobei der Flansch in der Vertiefung angeordnet ist und sich darin verschiebt, wenn der Katheteransatz (404) entlang der Längsachse des Mantelansatzes (402) repositioniert wird.

9. Vorrichtung (400) gemäß Anspruch 1, ferner umfassend ein Anschlagelement (522), das an einem von dem Katheteransatz (404) und dem Mantelansatz (402) gekoppelt ist, wobei der Katheteransatz (404) entlang der Längsachse des Mantelansatzes (402) zwischen einer eingeführten Stellung (424) und einer ausgefahrenen Stellung (426) positionierbar ist, wobei das Anschlagelement (522) zum Eingriff mit dem Feststellelement (516) gestaltet ist, um zu verhindern, dass der Katheteransatz (404) aus dem Katheteransatz-Aufnahmeraum (504) entfernt wird.

## Revendications

1. Appareil (400) pour faciliter le traitement intranasal de la fosse sphéno-palatine/ptérygo-platine d'un patient, l'appareil (400) comprenant, en combinaison:
un moyeu de gaine (402) présentant une surface extérieure (406) à l'opposé d'une surface intérieure (502), dans lequel la surface intérieure (502) définit un espace de réception de moyeu de cathéter (504);
un moyeu de cathéter (404) logé de façon coulissante à l'intérieur de l'espace de réception de moyeu de cathéter (504), dans lequel le moyeu de cathéter (404) peut être positionné le long d'un axe longitudinal du moyeu de gaine (402);
une gaine (420) couplée au moyeu de gaine (402) et un cathéter (412) couplé au moyeu de cathéter (404) et logé à l'intérieur de la gaine (420), dans lequel le cathéter (412) comprend une extrémité d'insertion (414) et une extrémité de couplage (506), l'extrémité d'insertion (414) présentant une courbure intrinsèque (416) par rapport à un axe longitudinal du cathéter (412), dans lequel le moyeu de cathéter (404) peut être positionné entre une position insérée (424) et une position étendue (426), et la gaine (420) redresse la courbure intrinsèque (416) du cathéter (412) lorsque le moyeu de cathéter (404) est positionné dans la position étendue (426),
un élément d'arrêt (516) sur un du moyeu de cathéter (404) et du moyeu de gaine (402); et
un élément d'engagement (520) sur l'autre du moyeu de cathéter (404) et du moyeu de gaine (402), dans lequel l'élément d'arrêt (516) engage de façon continue l'élément d'engagement (520) lorsque le moyeu de cathéter (404) est repositionné le long de l'axe longitudinal du moyeu de gaine (402) et dans lequel l'engagement entre l'élément d'arrêt (516) et l'élément d'engagement (520) empêche la rotation du moyeu de gaine (402) par rapport au moyeu de cathéter (404),
**caractérisé en ce que** le moyeu de cathéter (404) comprend une partie de manipulation (706), une partie d'insertion (708) et une partie de couplage (710), dans lequel la partie de manipulation (706) présente un diamètre accru par rapport à la partie d'insertion (708) et est positionnée entre la partie de couplage (710) et la partie d'insertion (708), dans lequel la partie d'insertion (708) commence à une surface d'arrêt (425) du moyeu de cathéter (404) et s'étend jusqu'à une extrémité de délivrance de traitement (704), et dans lequel la surface d'arrêt (425) du moyeu de cathéter (404) contacte le moyeu de gaine (402) pour arrêter une insertion supplémentaire du moyeu de cathéter (404) à l'intérieur de l'espace de réception de moyeu de cathéter (504) lorsque la partie d'insertion (708) du moyeu de cathéter (404) est entièrement insérée dans l'espace de réception de moyeu de cathéter (504).

2. Appareil (400) selon la revendication 1, comprenant en outre un indicateur d'orientation de rotation (408), l'indicateur d'orientation de rotation (408) identifiant une orientation de rotation de la courbure intrinsèque (416) du cathéter (412).

3. Appareil (400) selon la revendication 2, dans lequel l'indicateur d'orientation de rotation (408) comprend une nervure surélevée (432) s'étendant longitudinalement le long d'au moins un du moyeu de gaine (412) et du moyeu de cathéter (404).

4. Appareil (400) selon la revendication 1, dans lequel le moyeu de cathéter (404) comprend au moins un indicateur de profondeur (806) qui indique une position de l'extrémité d'insertion (414) du cathéter (412) par rapport à une extrémité d'introduction (505) de la gaine (420).

5. Appareil (400) selon la revendication 1, dans lequel l'extrémité d'insertion (414) présente une pointe (510) qui est incurvée de telle manière que la pointe (510) soit arrondie, la gaine comprenant une extrémité d'introduction (505) présentant un bord extrême (1302) qui est incliné vers un sommet (508) et dans lequel le sommet (508) est aligné avec un début (1304) de la courbe (1306) de la pointe arrondie (510) du cathéter (412) lorsque le moyeu de cathéter (404) est positionné dans la position étendue (426).

6. Appareil (400) selon la revendication 5, dans lequel une transition entre le sommet (508) et le début (1304) de la courbe (1306) de la pointe arrondie (510) du cathéter (412) est continue lorsque le moyeu de cathéter (404) est positionné dans la position étendue (426) .

7. Appareil (400) selon la revendication 5, dans lequel la pointe (510) de l'extrémité d'insertion du cathéter (412) est en forme de bulbe.

8. Appareil (400) selon la revendication 1, dans lequel l'élément d'arrêt (516) comprend une bride couplée à et s'étendant perpendiculairement à partir d'une parmi une surface extérieure (18) du moyeu de cathéter (404) et une surface intérieure (502) du moyeu de gaine (402), dans lequel l'élément d'engagement (520) comprend un retrait s'étendant longitudinalement le long de l'autre parmi la surface intérieure (502) du moyeu de gaine (402) et la surface extérieure (18) du moyeu de cathéter (404), dans lequel la bride est positionnée à l'intérieur et se déplace le long du retrait lorsque le moyeu de cathéter (404) est repositionné le long de l'axe longitudinal du moyeu de gaine (402).

9. Appareil (400) selon la revendication 1, comprenant en outre un élément d'arrêt (522) couplé à un du moyeu de cathéter (404) et du moyeu de gaine (402), dans lequel le moyeu de cathéter (404) peut être positionné le long de l'axe longitudinal du moyeu de gaine (402) entre une position insérée (424) et une position étendue (426), dans lequel l'élément d'arrêt (522) est configuré de façon à engager l'élément d'arrêt (516) afin d'empêcher le moyeu de cathéter (404) d'être retiré hors de l'espace de réception de moyeu de cathéter (504).
